# EUROPÄISCHE PATENTSCHRIFT

(11) **EP 3 865 052 B1**
(45) Veröffentlichungstag und Bekanntmachung des Hinweises auf die Patenterteilung: **02.09.2026**
(21) Anmeldenummer: 21156511.4
(22) Anmeldetag: 11.02.2021
(51) Int. Cl.: A61B 5/00, G01J 3/10

(54) **MESSSYSTEM UND MESSVERFAHREN ZUR BESTIMMUNG VON SONNENSCHUTZFAKTOREN VON SONNENSCHUTZMITTELN**
MEASURING SYSTEM AND MEASURING METHOD FOR DETERMINING THE SUN PROTECTION FACTOR OF A SUNSCREEN
SYSTÈME DE MESURE ET PROCÉDÉ DE MESURE POUR DÉTERMINER LE FACTEUR DE PROTECTION D'UNE CRÈME SOLAIRE

(30) Priorität: 11.02.2020 DE 102020103490
(43) Veröffentlichungstag der Anmeldung: 18.08.2021
(73) Patentinhaber: Courage + Khazaka Electronic GmbH, 50829 Köln (DE)
(72) Erfinder: Reble, Carina, 10829 Berlin (DE); Wiora, Georg, 50733 Köln (DE)
(74) Vertreter: Dantz, Dirk

(56) Entgegenhaltungen:
- WO-A1-2017/067545
- WO-A1-2020/232047
- DE-A1- 102014 003 470
- US-A1- 2018 202 927
- GEORG WIORA ET AL: "Development of a fast non-invasive in vivo measurement of UVA-PF and SPF with new Diffuse Reflectance Spec- troscopy device", 30 September 2019 (2019-09-30), XP055708127, Retrieved from the Internet <URL:https://www.skinobs.com/news/wp-content/uploads/2019/11/Development-of-a-fast-non-invasive-in-vivo-measurement-full-paper.CK_.pdf> [retrieved on 20200623]

## Beschreibung

Die Erfindung beschreibt ein Messsystem und Verfahren zur Qualifizierung von Kosmetika und Sonnenschutzmitteln, für die ein Sonnenschutzfaktor (SPF) oder ein noch neu zu definierender Schutzfaktor angegeben wird.

### Stand der Technik

In der DE 10 2014 003 470 A1 wird ein Messsystem zur Bestimmung von Sonnenschutzfaktoren von Sonnenschutzmitteln mit einer Strahlquelleneinrichtung wobei die Strahlquelleneinrichtung zwei oder mehr separate Strahlquellen aufweist, einem Spektrometer und einer Steuerungseinrichtung, wobei die Wellenlangenspektren des von mindestens zwei der separaten Strahlquellen abgestrahlten Strahls verschieden sind, sowie ein entsprechendes Verfahren zur Bestimmung der Sonnenschutzfaktoren.

Die WO 2017/067 545 A1 beschreibt ein Messsystem und ein Messverfahren, dass der Ermittlung eines Schutzfaktors für Licht dient, beispielsweise für Kosmetika und Sonnenschutzmitteln, für die ein Sonnenschutzfaktor (SPF) angegeben wird, indem zwei Messungen der Lichtrückstreuung an der Hautoberfläche in vivo oder in vitro oder auch an Hautmodellen (tierische Hautmodelle oder künstliche Hautmodelle) vor und nach Aufbringen des Strahlungsschutzmittels auf die Haut erfolgen und der Lichtschutzfaktor daraus ermittelt wird. Im Gegensatz zu bisher standardisierten Verfahren wird für den Strahlengang des Messverfahrens ein Abstand zwischen Beleuchtungsfläche und Detektionsfläche auf der Haut festgelegt. Der Lichtschutzfaktor kann nach bisherigen Standards ermittelt werden oder für weitere Wellenlängen bzw. Wellenlängenbereiche (z.B. UVA, VIS, NIR, IR).

Die US 2018/0202927 A1 beschreibt einen Sonnenschutzdetektor zur Verwendung mit tragbaren Geräten. Eine Variante eines Sonnenschutzdetektors umfasst ein Beleuchtungssystem, das so konfiguriert ist, dass es einen Bereich der Haut mit ultraviolettem und infrarotem Licht beleuchtet, sowie ein Sensorsystem, das so konfiguriert ist, dass es die Menge an ultraviolettem und infrarotem Licht erfasst, die von dem Bereich der Haut reflektiert wird. Der Sonnenschutzdetektor ist so konfiguriert, dass er die vom Sensorsystem gesammelten Daten analysiert, um eine Benachrichtigung an den Benutzer zu generieren, ob dieser Sonnenschutz auftragen sollte. In einer Ausführungsform umfasst das Beleuchtungssystem eine erste LED, die Licht im UVB Bereich (von etwa 290 nm bis etwa 320 nm) emittiert und eine zweite LED, die Licht im UVA Bereich (von etwa 320 nm bis etwa 400 nm) emittiert. Die Lichtquellen können gepulst betrieben werden.

Die bisherigen durch die Behörden der europäischen Union (EU) und der amerikanischen Food and Drug Administration (FDA) zugelassenen Methoden für die Bestimmung des SPF sind alle schädigend für den beteiligten Probanden, indem sie ein Erythem, also eine durch Licht hervorgerufene Entzündungsreaktion der Haut hervorrufen (COLI PA - European Cosmetic, Toiletry and Perfumery Association: Colipa SPF Test Method 94/289, 1994; ISO-Standards 24442, 24443, 24444). Daher haben sowohl die FDA als auch die EU mehrfach darauf hingewiesen, dass künftige Forschungsaktivitäten auf neue Methoden zur Charakterisierung der Schutzwirkung von Sonnenschutzmitteln gerichtet werden müssen, um Spätfolgen für die Probanden zu vermeiden (European Commission, Standardisation Mandate Assigned To CEN Concerning Methods For Testing Efficacy Of Sunscreen Products, M/389 EN, Brüssels, 12 July 2006).

Diese Aufgabe soll mit dieser Erfindung wahrgenommen werden. Die bestehenden Verfahren sind in verschiedenen Fundstellen definiert:
I. In Normen und Vorschriften definierte Verfahren:
   a. ISO 24444 definiert ein Verfahren zur In-vivo-Bestimmung des SPF. Grundlage des Verfahrens ist die Erzeugung von Erythemen auf der Haut von Probanden durch Strahlung im UVB-Bereich. Daher ist das Verfahren schädigend. Um die Abhängigkeit des Ergebnisses von interindividuellen Variationen der Hauteigenschaften zu verringern, muss das Verfahren an mehreren Probanden durchgeführt werden.
   b. ISO 24443 definiert ein In-vitro-Verfahren zur Bestimmung des UVA-Schutzfaktors (UVAPF). Das Sonnenschutzmittel wird auf eine Kunststoffplatte aufgetragen, so dass ein Transmissionsspektrum des Sonnenschutzmittels gemessen werden kann. Aufgrund nicht kontrollierbarer Schwankungen der Prozedur wird das Transmissionsspektrum durch eine Skalierung an das Ergebnis des Erythem-Tests nach ISO 24444 angepasst und ist damit von dessen Durchführung abhängig. Die verwendete Kunststoffplatte ist mit einer aufgerauten Oberfläche ein relativ unrealistisches Hautmodell.
   c. ISO 24442 definiert ein In-vivo-Verfahren, in dem der UVA-Schutzfaktor mittels der minimalen UVA-Dosis zur Erzeugung einer irreversiblen Pigmentierung (Sonnenbräune) der Haut bestimmt wird. Auch dieses Verfahren bedingt eine Veränderung der Haut des Probanden.
   d. FDA Final Rules 201 1 , ursprünglich veröffentlicht im Federal Register vom 27. August 2007 (72 FR 49070) und kodifiziert als Broad Spectrum Test (21 CFR 201.327(j)) und Sun Protection Factor (SPF) Test (21 CFR 201.327(i) bzw. in einer neueren Version als 21 CFR 201.352 (http://www.ecfr.gov/cgi-bin/text- idx? SID=5555a0dd8b6d83a8570676d9a44bb6ef&mc=true&node=pt21.5.352&rgn=div5 )
II. Patentierte Verfahren:
   a. DE 198 28 497 A1 beschreibt ein Verfahren, in dem wie in der ISO 24444 bei Probanden durch UV-Bestrahlung der Haut Erytheme erzeugt werden. Diese werden im Gegensatz zur ISO 24444 durch Reflexionsspektroskopie detektiert. Das Verfahren ist damit ebenfalls schädigend. Die optische Wirkung (Schutz) des Sonnenschutzmittels wird nicht mit direkten optischen Messungen erfasst, sondern über eine biologische Reaktion des Körpers.
   b. In DE 10 2004 020 644 A1 wird ein Verfahren beschrieben, in dem die Erzeugung von Radikalen durch UV-Belichtung in vivo mittels Elektronenspinresonanz (ESR) quantitativ gemessen wird. Auch hier wird die optische Wirkung des Sonnenschutzmittels nur indirekt erfasst. Zudem ist die Messung der ESR technisch aufwändig und erfordert relativ große, stationäre Geräte (Tischgeräte). Auch sind sie empfindlich auf Störungen durch Hochfrequenz-Einstrahlungen oder schnelle temporäre Magnetfeldänderungen, wie z.B. von elektrischen Schaltvorgängen.
III. Veröffentlichungen:
   a. In Bendova H, et al., Toxicology in vitro (2007), 1268-1275 werden Verfahren der Transmissionsspektroskopie unter Verwendung verschiedener Folien als Hautmodelle verglichen. Die ermittelten Schutzfaktoren hängen stark von der verwendeten Folie ab. Eine signifikante Korrelation der Schutzfaktoren mit dem SPF aus der ISO 24444 wurde nicht gefunden.
   b. In Ruvolo E, Kollias N, Cole C, Photodermatol Photoimmunol Photomed (2014), 30: 202- 211 wird ein Verfahren vorgestellt, in dem eine UVB-Transmissionsmessung mit Kunststoffsubstraten und eine In-vivo-Rückstreumessung an der Haut im UVA-Bereich kombiniert werden. Die Transmissionsmessung wird durch Skalierung an die UVA-Rückstreumessung angepasst, wodurch eine gute Übereinstimmung mit dem In-vivo-Test des SPF nach ISO 24444 erreicht wird. Die Messanordnung für die UVA-Rückstreumessung enthält ein Faserbündel, das auf der Haut aufgesetzt wird. Die Messung erfolgt über eine Vielzahl von Beleuchtungs- und Detektionsfasern mit unterschiedlichen Abständen zueinander. Detektiert wird die Summe der Lichtleistungen aus den Detektionsfasern, daher ist kein definierter Abstand zwischen Beleuchtungsfläche und Detektionsfläche gegeben, sondern eher eine Summierung aller Abstände. In dem erfindungsgemäßen Verfahren wird die Rückstreuung mit mehreren wohldefinierten Abständen gemessen.
   c. In Sohn M, Korn V, Imanidis G, Skin Pharmacol Physiol, (2015), 28: 31-41 wird Haut vom Schweineohr als Substrat für In-vitro-Messungen der Transmission erprobt. Im Vergleich zur Messung mit standardisierten Kunststoffträgern ergibt sich eine bessere Korrelation zum in vivo ermittelten SPF. Dies zeigt, dass ein realistisches Hautmodell für die Bestimmung von Schutzfaktoren wesentlich ist. Die Transmissionsmessung erfolgte mit/ohne Sonnenschutzmittel. Der Fehler einer solchen Messung ist aber stark von der genutzten Schichtdicke (Dicke der separierten Schicht des Schweineohrs) und der Oberflächenrauigkeit relativ zur Änderung durch die (identische, normierte) Menge an Sonnenschutzmittel abhängig und wird in den Messungen der Publikation nur gegen den per ISO 24444:2010 in vivo ermittelten SPF bewertet, nicht untereinander. Auch wurde nur ein Typ von Sonnenschutzmittel (Öl-in-Wasser) genutzt, der durch die Öl-Tröpfchenbildung spezifisch hohe Streueigenschaften hat, wodurch die Oberflächenänderungen weniger stark wirksam werden.

Der Einsatzbereich der bisherigen Verfahren mit UVB-Strahlung (solar Simulator, also "Sonnensimulator" mit vorgegebener wellenlängenspezifischer Intensität zwischen 290 bis 400 nm entsprechend der Sonnenbestrahlung auf Meereshöhe) und mit eingeschränkten Möglichkeiten mit UVA-Strahlung (Erythembildung erfolgt nicht wie bei UVB, höhere Eindringtiefe als UVB in die Haut = größeres Volumen) soll mit der erfindungsgemäßen Lösung uneingeschränkt auf das UVA, UVB, den sichtbaren und nahinfraroten bzw. infraroten Bereich für die Ermittlung entsprechender Lichtschutzfaktoren ausgedehnt werden.

### Nachteile bisheriger Verfahren

Erhöhte Dosen von UV-Strahlung können das Gewebe und zelluläre Bestandteile schädigen. Hautalterung und schlimmstenfalls Hautkrebs sind bekanntermaßen die Folgen. Seit Jahrzehnten ist eine steigende Zahl an Neuerkrankungen von Hautkrebs zu beobachten, die in Deutschland aktuell bei ca. 20000 Fällen pro Jahr liegt. Hauptursache ist eine wiederkehrende intensive UV-Belastung, wie sie in Sommerurlauben vorkommt, insbesondere in der Kindheit und Jugend.

Bestehende Verfahren zur Bewertung von Sonnenschutzmitteln sind unzulänglich, da sie entweder invasiv durch Erythembildung am Probanden getestet oder unphysiologisch an Kunststoffträgern als Hautmodell erprobt werden.

Die heutige In-vivo-SPF-Bestimmung weist eine Reihe von Unzulänglichkeiten auf. Diese Bestimmung bezieht sich nur auf eine spontane biologische Wirkung (erzwungener Sonnenbrand), die durch UVB-Strahlung ausgelöst wird. Heute weiß man jedoch, dass auch die UVA-Strahlung zu starken Hautschädigungen bis hin zum Hautkrebs führen kann. Darüber hinaus ist die Bestimmung des SPF ein invasives Verfahren, da eine Schädigung in Form des Sonnenbrandes bei den Probanden hervorgerufen wird. Daher haben sowohl die Amerikanische Food and Drug Administration (FDA) als auch die Europäische Union mehrfach darauf hingewiesen, dass künftige Forschungsaktivitäten auf neue Methoden zur Charakterisierung der Schutzwirkung von Sonnenschutzmitteln gerichtet werden müssen, um Spätfolgen für die Probanden zu vermeiden. Der In-vivo-SPF kann nur im UVB ermittelt werden, wohingegen langfristige Schädigungen auch durch andere Spektralbereiche auftreten.

Es ist daher Aufgabe der Erfindung, ein Verfahren und eine Vorrichtung bereitzustellen, die insbesondere die Belastung infolge der Einstrahlung auf den Messkörper verringern und zugleich qualitativ hochwertige Analyseergebnisse bereitstellen.

Die Aufgabe wird mittels des Messsystems gemäß Anspruch 1 gelöst. Vorteilhafte Ausführungen der Erfindung sind in den Unteransprüchen dargelegt.

Das erfindungsgemäße Messsystem zur Bestimmung von Sonnenschutzfaktoren weist eine Strahlquelleneinrichtung auf, die zwei oder mehr separate Strahlquellen aufweist. Außerdem weist das Messsystem ein Spektrometer zur Detektion des von einem Probenkörper reflektierten Lichts sowie eine Steuerungseinrichtung auf. Erfindungsgemäß sind die Wellenlängenspektren von mindestens zwei der von den separaten Strahlquellen abgestrahlten Strahlen voneinander unterschiedlich.

Das erfindungsgemäße Messsystem ermöglicht durch die gezielte Ansteuerung der Strahlquellen eine gezielte Anpassung des Gesamtspektrums auf verschiedene Anwendungen. Durch gezielte Überlagerung der einzelnen Spektralbereiche der Strahlquellen wird außerdem eine gleichmäßige Ausleuchtung sowohl im UVA-Wellenlängenbereich von 380 nm bis 315 nm als auch im UVB-Wellenlängenbereich von 315 nm bis 280 nm erreicht und damit auch die Wählbarkeit einer maximalen Strahlungsdosis, entweder in Form einer individuellen Dosis (z. B. 0,1 MED) oder eines Grenzwerts erreicht. In Summe wird so die Strahlungsdosis für einen Probanden minimiert. Nicht zuletzt kann die Lichtquelle auch für andere Messaufgaben verwendet werden, z.B. zur Messung der Photodegradation von Sonnenschutzprodukten, wofür ein sonnenähnliches Spektrum nötig ist. Erfindungsgemäß umfasst die Lichtquelle acht LEDs in einem Wellenlängebereich von 290 nm bis 400nm mit den Mittelwellenlängen im Bereich von 295nm, 310nm.325nm, 340nm, 355nm, 365nm, 385nm, und 395nm mit typischen spektralen Breiten der (FWHM) von 10-20nm.

Durch die puls-und regelbaren Strahlquellen ergibt sich die Möglichkeit einer flexiblen zeitlichen Steuerung der Beleuchtung sowie die Möglichkeit der Intensitätsregelung über Pulswellenmodulation (PWM). Die Kombination von mehreren einzeln steuerbarer polychromatischer Strahlquellen und einem Spektrometer ermöglicht es einerseits ein kontinuierliches Spektrum zu messen und andererseits eine Fluoreszenz- und Streulichtfilterung durchzuführen. Hierbei ist das Messsystem optional so ausgelegt, dass die einzelnen Strahlquellen der Strahlquelleneinrichtung getrennt und separat ansteuerbar sind, d.h. für jede einzelne Strahlquelle unterschiedliche Parameter und Einstellungen festlegbar sind. Diese Parameter umfassen Strom, Intensität (unabhängig von Strom über PWM) und Temperatur. Die einzelnen Strahlquellen des Messsystems emittieren zudem zueinander unterschiedliche Wellenlängenspektren. Es handelt sich vorzugsweise um polychromatische Lichtquellen, wie beispielsweise UV-LEDs. Sie sind pulsbar und regelbar hinsichtlich der von ihnen abgestrahlten Intensität. Gemäß der Erfindung sind die Strahlquellen dynamisch ansteuerbar, d.h. die Parameter werden abhängig von den Messdaten (z.B. Remission der Haut) dynamisch während einer Messsequenz angepasst. Das ermöglicht eine bessere Ausnutzung der begrenzten Dynamik des Detektors.

In einer weiteren Gestaltung der Erfindung ist das Spektrometer mittels der Steuerungseinrichtung steuerbar, außerdem sind die vom Spektrometer gemessenen Signale mittels der Steuerungseinrichtung verarbeitbar. Das Detektionsfenster und die Auflösung des Spektrometers wird mittels der Steuerungseinrichtung festgelegt, die detektierten Signale gespeichert, aufbereitet (z.B. verstärkt) und dargestellt.

Gemäß der Erfindung sind die einzelnen Strahlquellen der Strahlquelleneinrichtung separat ansteuerbar. Dadurch wird eine gezielte Ansteuerung der Strahlquellen und eine gezielte Anpassung des Gesamtspektrums auf verschiedene Anwendungen ermöglicht. Durch gezielte Überlagerung der einzelnen Spektralbereiche der Strahlquellen wird außerdem eine gleichmäßige Ausleuchtung erreicht und damit auch die Wählbarkeit einer maximalen Strahlungsdosis oder eines Grenzwerts erreicht. In Summe wird so die Strahlungsdosis für einen Probanden minimiert. Nicht zuletzt kann die Lichtquelle auch für andere Messaufgaben verwendet werden, z.B. zur Messung der Photodegradation von Sonnenschutzprodukten, wofür ein sonnenähnliches Spektrum nötig ist.

In einer weiteren Ausführung der Erfindung weist das Messsystem eine Strahlquellensteuerung auf, mittels der die einzelnen Strahlquellen der Strahlquelleneinrichtung separat ansteuerbar sind.

In einer weiteren Ausbildung der Erfindung weist das Messsystem eine Strahlquellensteuerung auf, mittels der die Strahlquelleneinrichtung ansteuerbar ist.

In einer weiteren Ausgestaltung der Erfindung ist mittels der Strahlquellensteuerung die Wellenlänge, Belichtungszeit und/oder die Intensität der von den einzelnen Strahlquellen ausgesandten Strahlung separat steuerbar. Dadurch wird eine gezielte Ansteuerung der Strahlquellen und eine gezielte Anpassung des Gesamtspektrums auf verschiedene Anwendungen ermöglicht. Durch definierte Überlagerung der einzelnen Spektralbereiche der Strahlquellen wird außerdem eine gleichmäßige Ausleuchtung erreicht und damit auch die Wählbarkeit einer maximalen Strahlungsdosis oder eines Grenzwerts erreicht. In Summe wird so die Strahlungsdosis für einen Probanden minimiert. Nicht zuletzt kann die Lichtquelle auch für andere Messaufgaben verwendet werden, z.B. zur Messung der Photodegradation von Sonnenschutzprodukten.

In einer weiteren Gestaltung der Erfindung ist die Strahlquellensteuerung separat von der Steuerungseinrichtung angeordnet. Die Steuerungseinrichtung ist üblicherweise ein PC oder Notebook-Computer mit entsprechendem Computerprogramm und mit der Strahlquellensteuerung über eine Datenleitung verbunden.

In einer weiteren Ausführung der Erfindung ist die Strahlquellensteuerung durch die Steuerungseinrichtung steuerbar. Die Steuerungseinrichtung ist üblicherweise ein PC oder Notebook-Computer mit entsprechendem Computerprogramm und mit der Strahlquellensteuerung über eine Datenleitung verbunden. Die Steuerungseinrichtung steuert über die Strahlquellensteuerung den Wellenlängenbereich, die Belichtungszeit und/oder die Intensität des von der Strahlquelleneinrichtung abgegebenen Lichts.

In einer weiteren Ausbildung der Erfindung ist die Strahlquellensteuerung mit dem Spektrometer verbunden und dafür geeignet und vorgesehen, Signale des Spektrometers zu empfangen und/oder Steuerbefehle an das Spektrometer zu senden. Das Detektionsfenster und die Auflösung des Spektrometers wird mittels der Strahlquellensteuerung festgelegt, die detektierten Signale gespeichert und aufbereitet (z.B. verstärkt).

Die Aufgabe wird ebenfalls mittels des erfindungsgemäßen Verfahrens gemäß Anspruch 8 gelöst.

Das erfindungsgemäße Verfahren zur Bestimmung von Sonnenschutzfaktoren weist drei Verfahrensschritte auf: Im ersten Verfahrensschritt wird eine erste Strahlquelle von einer mehrere Strahlquellen aufweisenden Strahlquelleneinrichtung angesteuert. Im zweiten Verfahrensschritt wird eine zweite Strahlquelle von einer mehrere Strahlquellen aufweisenden Strahlquelleneinrichtung angesteuert. Im dritten Verfahrensschritt wird ein Messkörper mit der Strahlung eines Gesamtspektrums aus der ersten und zweiten Strahlquelle bestrahlt. Erfindungsgemäß unterscheidet sich der Ansteuerungsbefehl der ersten Strahlquelle zur Bestrahlung des Messkörpers vom Ansteuerungsbefehl der zweiten Strahlquelle.

Dieses Verfahren hat zahlreiche Vorteile gegenüber dem Stand der Technik. So wird bei dem erfindungsgemäßen Verfahren nur eine kleine Lichtdosis unterhalb einer MED (minimale Erythem-Dosis; individuell für Hauttypen) bzw. unterhalb der maximal zulässigen Bestrahlung (MZB-Wert; für UV und auch andere Wellenlängenbereiche) auf den Messkörper eingestrahlt. Das Verfahren ist aufgrund dieser geringen Lichtdosen auch für einen schädigungsfreien In-vivo-Einsatz geeignet. Dies hat den Vorteil, dass die identischen physiologischen Bedingungen bei der SPF-Testung und bei der Anwendung in der Sonne vorliegen. Das erfindungsgemäße Verfahren wird zudem nicht-invasiv angewendet. Weiterhin wird bei dem Verfahren die Lichtausbreitung in der Haut berücksichtigt und hierdurch eine erhöhte Messgenauigkeit erreicht. Die Berücksichtigung der physiologischen Eigenschaften durch ein realistischeres Hautmodel führen zu einer Verbesserung der Bestimmung des Sonnenschutzfaktors im UVA und im sichtbaren Spektralbereich. Weiterhin ist eine Angleichung von In-vivo- (menschliche Haut) und In-vitro-Test möglich. Zudem ist das Verfahren für einen sehr großen Wellenlängenbereich nutzbar, nicht limitiert durch Lampenspektren, Erythem-Wirkspektrum, Reaktionen des Messkörpers, o.ä..

Die einzelnen Strahlquellen der Strahlquelleneinrichtung werden in einer alternativen Ausgestaltung der Erfindung getrennt und separat angesteuert, d.h. für jede einzelne Strahlquelle werden unterschiedliche Parameter und Einstellungen festgelegt. Diese Parameter umfassen Strom, Intensität (unabhängig von Strom über PWM) und Temperatur. Die einzelnen Strahlquellen des Messsystems emittieren zudem zueinander unterschiedliche Wellenlängenspektren. Es handelt sich hierbei vorzugsweise um polychromatisches Licht, das aus polychromatischen Lichtquellen wie beispielsweise UV-LEDs abgestrahlt wird. Die Strahlquellen können gepulst betrieben werden. Die Strahlquellen können weiterhin hinsichtlich der von ihnen abgestrahlten Intensität und des Wellenlängenbereichs geregelt werden. In einer Weiterführung der Erfindung werden die Strahlquellen dynamisch angesteuert. Eine Anpassung des der Detektion ist daher nicht erforderlich. Durch ein solches Verfahren werden Streulicht- und Fluoreszenzanteile der von der Probe reflektierten Strahlung gefiltert und die begrenzte Dynamik der Detektion besser genutzt.

Das erfindungsgemäße Verfahren bietet auch die Möglichkeit, den Spektralbereich für die Messung zu erweitern, da im Wesentlichen nur bei UVB eine Erythembildung induziert wird und dieser Messbereich für die Bestimmung des Sonnenschutzfaktors von besonderer Bedeutung ist. Infolge der geringen auf den Messkörper eingestrahlten Lichtdosis sind keinerlei Einschränkungen hinsichtlich des Messortes zu beachten. Selbst Messungen an der empfindlichen Kopfhaut sind möglich. Weiterhin wird auch der Einfluss von Substanzen auf die erfindungsgemäße Messung vermieden, die eine Hautrötung bzw. UV-induziertes Erythem verursachen. Das erfindungsgemäße Verfahren ist einfacher und aussagekräftiger als die bisher bekannten Verfahren und mit geringeren Kosten verbunden.

In einer weiteren Ausgestaltung der Erfindung wird ein Referenzspektrum einer Strahlquelle erfasst. Das Referenzspektrum wird mittels einer Standardprobe ermittelt, um Wellenlängenbereich und Intensität des von der Strahlquelle ausgesandten Lichts zu ermitteln.

In einer weiteren Ausführung der Erfindung wird aus dem Referenzspektrum der einen Strahlquelle eine Zentralwellenlänge der einen Strahlquelle für ein spektrales Detektionsfenster für die eine Strahlquelle bestimmt. Das Referenzspektrum wird mittels einer Standardprobe ermittelt, um Wellenlängenbereich und Intensität des von der Strahlquelle ausgesandten Lichts zu ermitteln. Aufgrund dieser Daten wird ein spektrales Detektionsfenster des Spektrometers bestimmt, für das das von der Strahlquelle ausgesandte Licht durchlässig ist.

In einer weiteren Gestaltung der Erfindung wird ein spektrales Fenster einer Strahlquelle bestimmt und/oder festgelegt. Um je nach Anforderung der Messung einen Wellenlängenbereich der Strahlquellen zu bestimmen, wird ein spektrales Fenster sowohl der Strahlquelle als auch des Spektrometers ermittelt bzw. festgelegt.

In einem weiteren Aspekt der Erfindung erfolgt die Bestimmung und/oder die Festlegung eines spektralen Fensters für das erste und das zweite Testspektrum getrennt.

In einer weiteren Ausführung der Erfindung wird das Spektrum einer Strahlquelle mathematisch gefiltert. Dazu wird das Spektrum z.B. mit einer Trapezfunktion und/oder einer geeigneten anderen Filterfunktion überlagert. Das Signal-zu-Rausch-Verhältnis wird so erhöht.

In einer weiteren Ausbildung der Erfindung erfolgt das mathematische Filtern des Spektrums einer Strahlquelle derart, dass das Signal eines gefilterten Spektrums außerhalb des festgelegten spektralen Fensters der einen Strahlquelle eine maximale gefilterte Intensität von 15% der maximalen ursprünglichen Intensität des ausgesandten Signals innerhalb des festgelegten spektralen Fensters aufweist. Bevorzugt ist die maximal gefilterte Intensität kleiner als 10%, besonders bevorzugt kleiner als 5 % der maximalen ursprünglichen Intensität. Alternativ oder zusätzlich kann das Signal eines gefilterten Spektrums außerhalb des festgelegten spektralen Fensters der einen Strahlquelle eine maximale Peak-Intensität von 15% der maximalen ursprünglichen Peak-Intensität des ausgesandten Signals innerhalb des festgelegten spektralen Fensters aufweist.

In einer Weiterbildung der Erfindung wird die Aufnahme eines Referenzspektrums für jede Strahlquelle separat ausgeführt. Bevorzugt werden hierbei auch die Verfahrensschritte Bestimmen der zentralen Wellenlänge des Referenzspektrums einer Strahlquelle und Bestimmung des mathematischen Filters einer Strahlquelle für jede Strahlquelle separat ausgeführt.

In einer weiteren Ausführung der Erfindung beinhaltet das Verfahren ein Aufnehmen eines ersten Testspektrums einer Strahlquelle. Aus dem ersten Testspektrum wird insbesondere die für die Messung zu verwendende Belichtungszeit und/oder die für die Messung zu verwendende Lichtleistung des von der Strahlquelle ausgesandten Lichts ermittelt.

In einem weiteren Aspekt der Erfindung wird das erste Testspektrum von der Bestrahlung eines unbehandelten Messkörpers aufgenommen. Das erste Testspektrum dient insbesondere zur Ermittlung der Belichtungszeit und/oder Lichtleistung des von der Strahlquelle ausgesandten Lichts.

In einer weiteren Ausbildung der Erfindung beinhaltet das Verfahren ein Aufnehmen eines zweiten Testspektrums einer Strahlquelle. Aus dem zweiten Testspektrum wird insbesondere die für die Messung zu verwendende Belichtungszeit und/oder die für die Messung zu verwendende Lichtleistung des von der Strahlquelle ausgesandten Lichts ermittelt.

In einer weiteren Ausführung der Erfindung wird das zweite Testspektrum von der Bestrahlung eines mit einem Sonnenschutzmittel behandelten Messkörpers aufgenommen. Das zweite Testspektrum dient wie das erste Testspektrum insbesondere zur Ermittlung der Belichtungszeit und/oder Lichtleistung des von der Strahlquelle ausgesandten Lichts.

In einer weiteren Ausgestaltung der Erfindung werden die Aufnahme der ersten und/oder der zweiten Testspektren für jede Strahlquelle separat ausgeführt. Damit ist für jede Strahlquelle Intensität und Wellenlängenbereich bekannt bzw. festgelegt und das Signal-zu-Rausch-Verhältnis verbessert.

In einer weiteren Ausführungsform der Erfindung wird ein erstes Messspektrum aufgenommen. Bevorzugt kommen hierfür die zuvor in der oder den durchgeführten Referenz- und/oder Testspektren bestimmten Parameter, wie beispielsweise die Zentralwellenlänge, des mathematischen Filters, der Belichtungszeit und oder der Lichtleistung zur Anwendung.

In einer Weiterbildung des erfindungsgemäßen Verfahrens wird das erste Messspektrum von der Bestrahlung eines unbehandelten Messkörpers aufgenommen.

In einer weiteren Ausführungsform der Erfindung wird ein zweites Messspektrum aufgenommen. Bevorzugt kommen hierfür die zuvor in der oder den durchgeführten Referenz- und/oder Testspektren bestimmten Parameter, wie beispielsweise die Zentralwellenlänge, des mathematischen Filters, der Belichtungszeit und oder der Lichtleistung zur Anwendung.

In einer Weiterbildung des erfindungsgemäßen Verfahrens wird das erste Messspektrum von der Bestrahlung eines mit einem Sonnenschutzmittel behandelten Messkörpers aufgenommen.

In einer weiteren Gestaltung der Erfindung wird ein Gesamtspektrum aus den Messspektren von zwei oder mehr Strahlquellen zusammengesetzt. Je nach Anwendung, z.B. Messung eines Sonnenschutzfaktors und/oder Bestimmung von Fluoreszenzen, wird durch das erfindungsgemäße Verfahren gezielt ein Emissionsspektrum ausgewählt und für die Messung verwendet.

In einer weiteren Ausführung der Erfindung wird ein erstes Gesamtspektrum aus gefilterten Spektren aus den ersten Messspektren von zwei oder mehr Strahlquellen zusammengesetzt und/oder ein zweites Gesamtspektrum aus gefilterten Spektren aus den zweiten Messspektren von zwei oder mehr Strahlquellen zusammengesetzt.

In einem weiteren Aspekt der Erfindung erfolgt das Bestrahlen eines Messkörpers mit Strahlung eines Gesamtspektrums aus der ersten und der zweiten Strahlquelle zeitgleich. Dazu wird üblicherweise eine Stelle an der Innenseite des Unterarms oder des Rückens eines Probanden beaufschlagt. Dieser Messort 3 wird mit dem Messsystem 1 vermessen, indem Licht durch Aufsetzen einer Lichtleitfaser eingestrahlt wird. Die Einstrahlung erfolgt mit einer Intensität, die keine akute Schädigung in der Haut verursacht.

Ausführungsbeispiele des erfindungsgemäßen Messsystems und des erfindungsgemäßen Verfahrens sind in den Zeichnungen schematisch vereinfacht dargestellt und werden in der nachfolgenden Beschreibung näher erläutert.

Es zeigen:
- Fig. 1:: Ein Ausführungsbeispiel des erfindungsgemäßen Messsystems.
- Fig. 2:: Ein weiteres Ausführungsbeispiel des erfindungsgemäßen Messsystems.
- Fig. 3 a:: Draufsicht eines Ausführungsbeispiels einer Strahlquelleneinrichtung mit acht Strahlquellen.

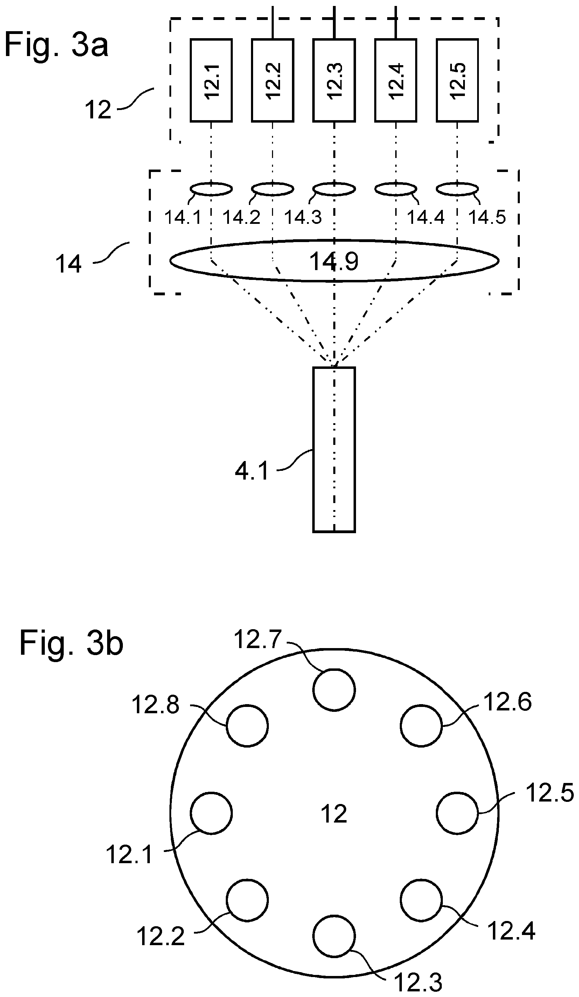

- Fig. 3 b:: Ansicht eines Ausführungsbeispiels einer Strahlquelleneinrichtung mit acht Strahlquellen entlang der Stahlrichtung.

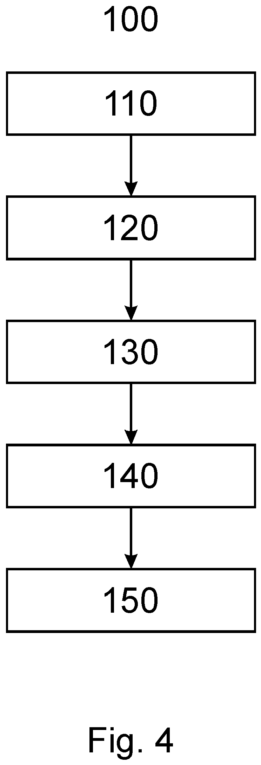

- Fig. 4:: Das erfindungsgemäße Verfahren zur Bestimmung von Sonnenschutzfaktoren.

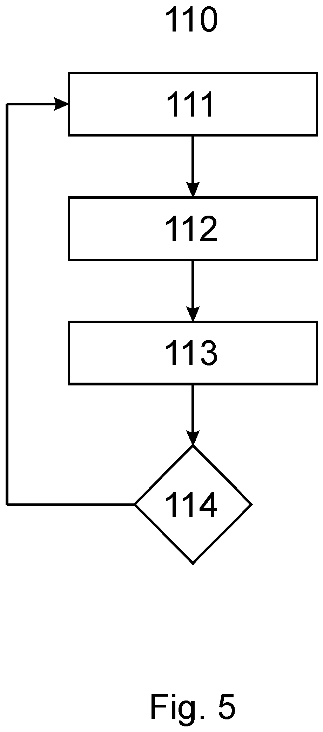

- Fig. 5:: Verfahren zur Aufnahme eines Referenzspektrums

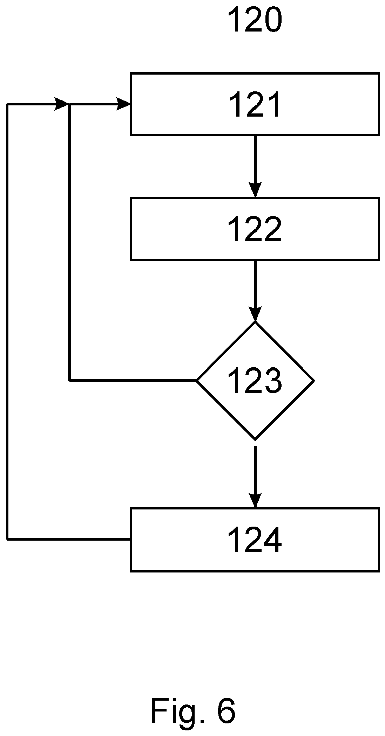

- Fig. 6:: Verfahren zur Aufnahme eines Testspektrums

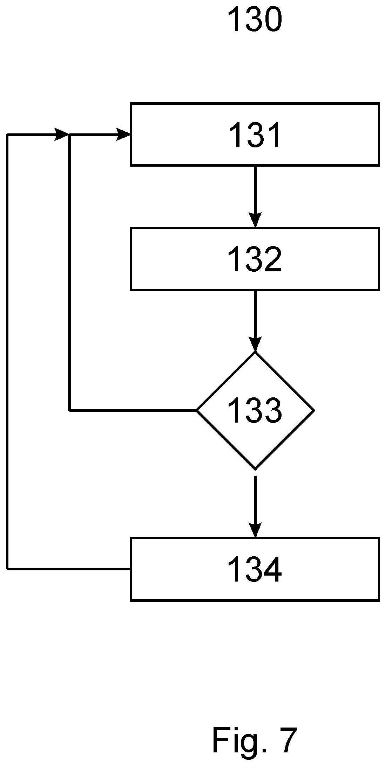

- Fig. 7:: Verfahren zur Aufnahme eines Messspektrums

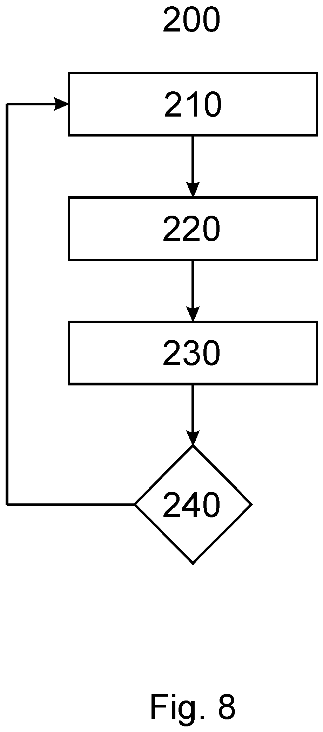

- Fig. 8.: Verfahren zur Aufnahme eines Referenz-, Test- oder Messspektrums

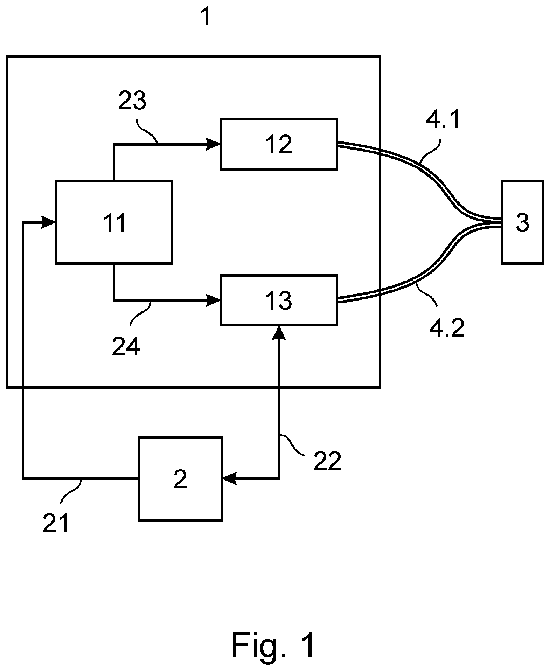

Fig. 1 zeigt schematisch ein Ausführungsbeispiel des erfindungsgemäßen Messsystems 1. Das Messsystem 1 weist eine Strahlquelleneinrichtung 12 mit mehreren Strahlquellen 12.1, 12.2, 12.3, 12.4, 12.5, 12.6, 12.7, 12.8 auf. Mittels eines Lichtleiters 4.1 wird das von der Strahlquelleneinrichtung 12 emittierte Spektrum in die Probe 3 eingeleitet, das von der Probe 3 reflektierte Licht gelangt über einen weiteren Lichtleiter 4.2 in das Spektrometer 13. Spektrometer 13 und Strahlquelleneinrichtung 12 sind über Datenleitungen 23, 24 mit einer Strahlquellensteuerung 11 verbunden, die ihrerseits über eine weitere Datenleitung 21 mit der extern angeordneten Steuerungseinrichtung 2 verbunden ist. Die Strahlquellensteuerung ist dafür vorgesehen die Strahlquellen dynamisch zu steuern.

Die Steuerungseinrichtung 2 ist üblicherweise ein PC oder Notebook-Computer mit entsprechendem Computerprogramm. Steuerungseinrichtung 2 und Spektrometer 13 sind ebenfalls über eine Datenleitung 22 miteinander verbunden.

Das Verfahren zur Bestimmung von Sonnenschutzfaktoren 100 beinhaltet vor der eigentlichen Messung zur Bestimmung eines Sonnenschutzfaktors eine Signaloptimierung der Strahlquelleneinrichtung 12. Damit wird eine gezielte Anpassung des Spektrums der Strahlquelleneinrichtung 12 auf verschiedene Anforderungen der Messung erzielt. Dazu wird ein Referenzspektrum einer Strahlquelle 12.1, 12.2, 12.3, 12.4, 12.5, 12.6, 12.7, 12.8 der Strahlquelleneinrichtung 12 aufgenommen. Aus diesem Referenzspektrum wird die Zentralwellenlänge und deren Intensität (Lichtleistung) bestimmt, die die Strahlquelle 12.1, 12.2, 12.3, 12.4, 12.5, 12.6, 12.7, 12.8 abstrahlt. Außerdem wird ein spektrales Fenster für die Strahlquelle 12.1, 12.2, 12.3, 12.4, 12.5, 12.6, 12.7, 12.8 festgelegt. Danach erfolgt die Aufnahme eines Testspektrums auf einen Messkörper 3, der nicht mit einem Sonnenschutzmittel behandelt ist. Das Testspektrum wird dann mathematisch derart gefiltert, dass das Testspektrum außerhalb des festgelegten spektralen Fensters entweder eine Intensität von 0 aufweist oder zur Einhaltung von Stetigkeitsbedingungen im Zusammengesetzten Spektrum eine Intensität aufweist, die nicht mehr wesentlich zum Ergebnis beiträgt. Hierzu kann eine Trapezfunktion oder eine Gauss-Funktion mit einem Exponenten größer 2 verwendet. Dieser geschilderte Prozess (Aufnahme Referenzspektrum, Festlegung eines spektralen Fensters, Aufnahme eines Testspektrums, Filterung) wird für jede Strahlquelle 12.1, 12.2, 12.3, 12.4, 12.5, 12.6, 12.7, 12.8 innerhalb der Strahlquelleneinrichtung 12 wiederholt. Zuletzt werden die Teilspektren nacheinander auf die Probe 3 eingestrahlt. Aus diesen Messspektren wird dann ein Gesamtspektrum zusammengesetzt, das zur Bestimmung des Sonnenschutzfaktors verwendet wird.

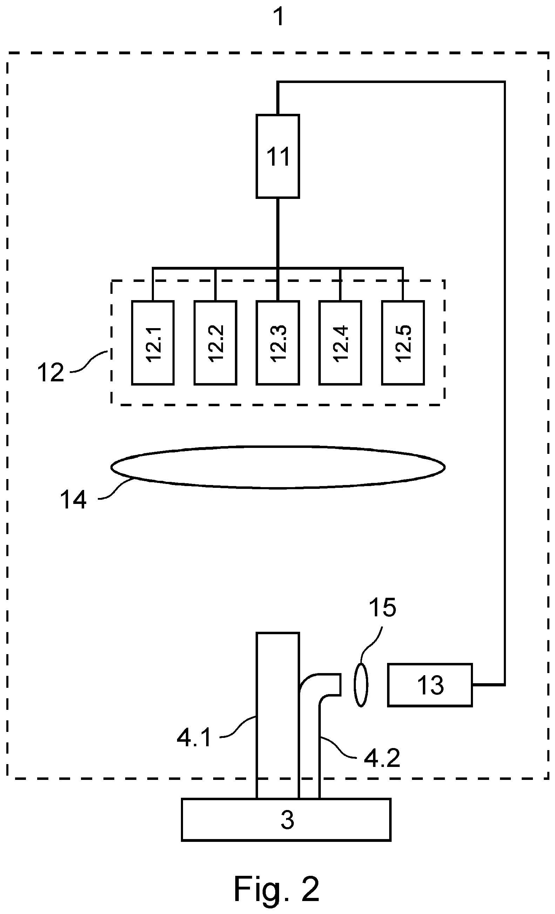

Eine detaillierte Ansicht eines Ausführungsbeispiels des erfindungsgemäßen Messsystems 1 zeigt Fig. 2. Das Messsystem 1 weist eine Strahlquelleneinrichtung 12 mit fünf Strahlquellen 12.1, 12.2, 12.3, 12.4, 12.5 auf. Die Strahlquellen 12.1, 12.2, 12.3, 12.4, 12.5 sind vorteilhafterweise LED's, die eine besonders lange Lebensdauer aufweisen. Die Spektralbereiche der einzelnen LED's unterscheiden sich derart voneinander, dass ein Spektralbereich insbesondere im UVA- (315-380 nm) und UVB-Bereich (280-315 nm) abgedeckt wird. Das von den Strahlquellen 12.1, 12.2, 12.3, 12.4, 12.5 ausgesendete Licht wird durch ein Linsensystem 14 gebündelt, in den Lichtleiter 4.1 eingekoppelt und auf die Probe 3 geleitet. Das von der Probe 3 reflektierte Licht gelangt über einen weiteren Lichtleiter 4.2 durch ein weiteres Linsensystem 15 in das Spektrometer 13. Spektrometer 13 und Strahlquelleneinrichtung 12 sind über die Datenleitung 24 mit der Strahlquellensteuerung 11 verbunden, die ihrerseits über eine weitere Datenleitung mit der extern angeordneten Steuerungseinrichtung 2 verbunden ist (nicht dargestellt, s. Fig. 1). Die Strahlquellensteuerung 11 steuert die Intensität und Wellenlängen der Strahlquelleneinrichtung 12 und empfängt dazu die Daten des Spektrometers 13.

Das Verfahren zur Bestimmung von Sonnenschutzfaktoren 100 beinhaltet vor der eigentlichen Messung zur Bestimmung eines Sonnenschutzfaktors eine Signaloptimierung der Strahlquelleneinrichtung 12.

Dazu wird ein Referenzspektrum einer Strahlquelle 12.1, 12.2, 12.3, 12.4, 12.5 der Strahlquelleneinrichtung 12 aufgenommen. Aus diesem Referenzspektrum wird die Zentralwellenlänge und deren Intensität (Lichtleistung) bestimmt, die die Strahlquelle 12.1, 12.2, 12.3, 12.4, 12.5 abstrahlt. Außerdem wird ein spektrales Fenster für die Strahlquelle 12.1, 12.2, 12.3, 12.4, 12.5 festgelegt. Danach erfolgt die Aufnahme eines Testspektrums auf einer Probe 3, die nicht mit einem Sonnenschutzmittel behandelt ist. Das Testspektrum wird dann mathematisch derart gefiltert, dass das Testspektrum außerhalb des festgelegten spektralen Fensters eine Intensität von 0 aufweist oder zur Einhaltung von Stetigkeitsbedingungen im Zusammengesetzten Spektrum eine Intensität aufweist, die nicht mehr wesentlich zum Ergebnis beiträgt. Hierzu kann eine Trapezfunktion oder eine Gauss-Funktion mit einem Exponenten größer 2 verwendet. Dieser geschilderte Prozess (Aufnahme Referenzspektrum, Festlegung eines spektralen Fensters, Aufnahme eines Testspektrums, Filterung) wird für jede Strahlquelle 12.1, 12.2, 12.3, 12.4, 12.5, 12.6, 12.7, 12.8 innerhalb der Strahlquelleneinrichtung 12 wiederholt. Zuletzt werden die Messspektren aufgenommen, indem die Teilspektren nacheinander auf die Probe 3 eingestrahlt werden. Aus diesen Messspektren wird dann ein Gesamtspektrum zusammengesetzt, das zur Bestimmung des Sonnenschutzfaktors verwendet wird.

Fig. 3 zeigt ein Ausführungsbeispiel einer Anordnung der Strahlquellen 12.1, 12.2, 12.3, 12.4, 12.5, 12.6, 12.7, 12.8 und des Linsensystems 14 in der Strahlquelleneinrichtung 12 zur Einkopplung des von den Strahlquellen 12.1, 12.2, 12.3, 12.4, 12.5, 12.6, 12.7, 12.8 ausgesandte Lichts in einen Lichtleiter 4.1 (Fig. 3 a).

Die Strahlquelleneinrichtung 12 weist acht Strahlquellen 12.1, 12.2, 12.3, 12.4, 12.5, 12.6, 12.7, 12.8, bevorzugt LED's, auf, die kreisförmig angeordnet sind (Fig. 3 b). Jede Strahlquelle 12.1, 12.2, 12.3, 12.4, 12.5, 12.6, 12.7, 12.8 kann mittels der Strahlquellensteuerung 11 einzeln angesteuert werden, d.h. einzelne Strahlquellen 12.1, 12.2, 12.3, 12.4, 12.5, 12.6, 12.7, 12.8 können aktiviert bzw. deaktiviert oder mehrere Strahlquellen 12.1, 12.2, 12.3, 12.4, 12.5, 12.6, 12.7, 12.8 sequentiell zur Beleuchtung verwendet werden, was die Untersuchung von wellenlängenabhängigen Effekten ermöglicht (z. B. Fluoreszenzerkennung).

Zur Bündelung des von den Strahlquellen 12.1, 12.2, 12.3, 12.4, 12.5, 12.6, 12.7, 12.8 erzeugten Lichtes ist vor jeder Strahlquelle 12.1, 12.2, 12.3, 12.4, 12.5, 12.6, 12.7, 12.8 eine Linse 14.1, 14.2, 14.3, 14.4, 14.5 angeordnet, die das von den Strahlquellen 12.1, 12.2, 12.3, 12.4, 12.5, 12.6, 12.7, 12.8 erzeugte Licht in die Linse 14.9 einstrahlt. Die Linse 14.9 bündelt das von den Strahlquellen 12.1, 12.2, 12.3, 12.4, 12.5, 12.6, 12.7, 12.8 erzeugte Licht auf einen Lichtleiter 4.1 und koppelt das Licht in diesen ein.

Das Verfahren zur Bestimmung von Sonnenschutzfaktoren beinhaltet vor der eigentlichen Messung zur Bestimmung eines Sonnenschutzfaktors eine Signaloptimierung der Strahlquelleneinrichtung 12.

Dazu wird ein Referenzspektrum einer Strahlquelle 12.1, 12.2, 12.3, 12.4, 12.5, 12.6, 12.7, 12.8 der Strahlquelleneinrichtung 12 aufgenommen. Aus diesem Referenzspektrum wird die Zentralwellenlänge und deren Intensität (Lichtleistung) bestimmt, die die Strahlquelle 12.1, 12.2, 12.3, 12.4, 12.5, 12.6, 12.7, 12.8 abstrahlt. Außerdem wird ein spektrales Fenster für die Strahlquelle 12.1, 12.2, 12.3, 12.4, 12.5, 12.6, 12.7, 12.8 festgelegt. Danach erfolgt die Aufnahme eines ersten und oder zweiten Testspektrums auf einem Messkörper 3, der nicht mit einem Sonnenschutzmittel behandelt ist. Das Testspektrum wird dann mathematisch derart gefiltert, dass das Testspektrum außerhalb des festgelegten spektralen Fensters eine Intensität von 0 aufweist oder zur Einhaltung von Stetigkeitsbedingungen im Zusammengesetzten Spektrum eine Intensität aufweist, die nicht mehr wesentlich zum Ergebnis beiträgt. Hierzu kann eine Trapezfunktion oder eine Gauss-Funktion mit einem Exponenten größer gleich 2 verwendet.

Dieser geschilderte Prozess (Aufnahme Referenzspektrum, Festlegung eines spektralen Fensters, Aufnahme eines Testspektrums, Filterung) wird für jede Strahlquelle 12.1, 12.2, 12.3, 12.4, 12.5, 12.6, 12.7, 12.8 innerhalb der Strahlquelleneinrichtung 12 wiederholt. Zuletzt werden die Teilspektren nacheinander auf die Probe 3 eingestrahlt und aus den gemessenen Messspektren wird dann ein Gesamtspektrum zusammengesetzt, das zur Bestimmung des Sonnenschutzfaktors verwendet wird.

Das erfindungsgemäße Verfahren 100 zur Bestimmung von Sonnenschutzfaktoren zeigt Fig. 4. Zu Beginn des Verfahrens 100 erfolgt die Durchführung einer Referenzmessung 110. Die Referenzmessung 110 umfasst die Aufnahme eines oder mehrerer Referenzspektren 111 zur Festlegung der spektralen Fenster der Strahlquellen 12.. Aus diesem Referenzspektrum wird die Zentralwellenlänge bestimmt 112 und ein mathematischer Filter ermittelt 113, um das spektrales Detektionsfenster (Wellenlängenbereich) für jede Strahlungsquelle zu bestimmen. In einem zweiten Verfahrensschritt erfolgt die Testmessung 120. Die Testmessung 120 umfasst die Aufnahme eines oder mehrerer Testspektren 121. Aus den Testspektren werden die Belichtungszeit und/oder die Lichtleistung der einzelnen Strahlungsquellen 12 ermittelt 122. Im Anschluss. daran werden die Probenmessungen durchgeführt 130. Im vierten Verfahrensschritt werden die aus den Probenmessungen resultierenden Teilspektren zu einem Gesamtspektrum zusammengesetzt 140. Aus den Gesamtspektren wird dann der Lichtschutzfaktor des Strahlungsschutzmittels ermittelt 150.

In einem Ausführungsbeispiel sind die Teilschritte des erfindungsgemäßen Verfahrens Durchführung der Referenzmessung 110, Durchführung der Testmessung 120 und Durchführung der Probenmessung 130 in den Fig. 5 - 7 dargestellt.

Bei der Durchführung der Referenzmessung wird für eine erste Strahlungsquelle eine Referenzmessung an einer Standardprobe durchgeführt 111. Für die erste Strahlungsquelle wird zunächst eine Zentralwellenlänge bestimmt 112. In einem nachfolgenden Schritt wird dann ein mathematischer Filter festgelegt 113. Dieser Verfahrensablauf wird für alle Strahlungsquellen 12.1, 12.2, 12.3, 12.4, 12.5, 12.6, 12.7, 12.8 wiederholt 114. Die Ermittlung des mathematischen Filters 114 erfolgt durch ein auf der Steuereinrichtung 2 ausgeführtes Softwareprogramm. Die mathematische Filterung erfolgt so, dass das gemessene Referenzspektrum außerhalb des festgelegten spektralen Fensters eine maximale gefilterte Intensität von 15% der maximalen ursprünglichen Intensität des Signals innerhalb des festgelegten spektralen Detektionsfensters aufweist oder zur Einhaltung von Stetigkeitsbedingungen im Zusammengesetzten Spektrum eine Intensität aufweist, die nicht mehr wesentlich zum Ergebnis beiträgt. Hierzu kann eine Trapezfunktion oder eine Gauss-Funktion mit einem Exponenten größer gleich 2 verwendet werden. Alternativ kann auch zuerst die Durchführung der Referenzmessung 110 für jede einzelne Strahlungsquelle 12.1, 12.2, 12.3, 12.4, 12.5, 12.6, 12.7, 12.8 erfolgen. Im Anschluss daran wird dann die zentrale Wellenlänge jeder Strahlungsquelle 12.1, 12.2, 12.3, 12.4, 12.5, 12.6, 12.7, 12.8 und die mathematische Filterung ermittelt.

Die Testmessung 120 (siehe Fig. 6) wird jeweils für jede Strahlungsquelle 12.1, 12.2, 12.3, 12.4, 12.5, 12.6, 12.7, 12.8 an einem unbehandelten Messkörper und einem mit einem Strahlungsschutzmittel behandeltem Messkörper 3 durchgeführt. Hierbei wird jeweils für jede Strahlungsquelle 12.1, 12.2, 12.3, 12.4, 12.5, 12.6, 12.7, 12.8 ein Testspektrum 121 von einem unbehandelten Messkörper 3 und einem mit einem Strahlungsschutzmittel behandeltem Messkörper 3 aufgenommen. Die Belichtungszeit wird hier sehr kurz gewählt, um die Strahlendosis möglichst gering zu halten. Sie dient der Ermittlung der optimalen Belichtungszeit und der optimalen Leistungsdosis und umfasst die Verfahrensschritte Aufnahme eines Testspektrums 121, Bestimmung der Belichtungszeit und/oder der Lichtleistung 122, Wiederholung der beiden zuvor genannten Verfahrensschritte für jede Strahlungsquelle 123. Für eine vollständige Testmessung erfolgt ein kompletter Durchgang für jede Strahlungsquelle 12.1, 12.2, 12.3, 12.4, 12.5, 12.6, 12.7, 12.8. Für die Testmessung 120 kommen bereits die zuvor in der Referenzmessung 110 ermittelten mathematischen Filter der einzelnen Strahlungsquellen 12.1, 12.2, 12.3, 12.4, 12.5, 12.6, 12.7, 12.8 bzw. die festgelegten spektralen Detektionsfenster zum Einsatz. Alternativ können auch erst die Testmessungen 121 für mehrere oder alle Strahlungsquellen 12.1, 12.2, 12.3, 12.4, 12.5, 12.6, 12.7, 12.8 durchgeführt werden. Die Belichtungszeit und/oder die Lichtleistung wird dann im Anschluss daran für jede Strahlungsquelle 12.1, 12.2, 12.3, 12.4, 12.5, 12.6, 12.7, 12.8 bestimmt. Hiernach erfolgt eine Abfrage 124, ob die jeweilige Aufnahme des Testspektrums 121 die Bestimmung der Belichtungszeit 122 für alle vorgesehenen Lichtquellen 123 sowohl für einen unbehandelten als auch einen behandelten Messkörper 3 erfolgt ist.

Die Probenmessung 130 (siehe Fig. 7) erfolgt unter der Verwendung der in der Referenzmessung 110 ermittelten zentralen Wellenlänge, Filtern und/oder spektralen Fenstern der einzelnen Strahlungsquellen 12.1, 12.2, 12.3, 12.4, 12.5, 12.6, 12.7, 12.8. Weiterhin kommen die in der Testmessung 120 ermittele Belichtungszeit und/oder die in der Testmessung ermittelte Lichtleistung für die einzelnen Strahlungsquellen 12.1, 12.2, 12.3, 12.4, 12.5, 12.6, 12.7, 12.8 in den Probenmessungen 130 zum Einsatz. Die Probenmessung 130 beinhaltet die Aufnahme von Probenspektren 131 mehrerer Strahlungsquellen 12.1, 12.2, 12.3, 12.4, 12.5, 12.6, 12.7, 12.8 eines unbehandelten und eines mit einem Strahlungsmittel behandelten Messkörpers 3. Hierbei werden Probenspektren von beiden Messkörpern 3 jeweils für jede der verwendeten Strahlungsquellen 12.1, 12.2, 12.3, 12.4, 12.5, 12.6, 12.7, 12.8 separat aufgenommen. Die Probenspektren der einzelnen Strahlungsquellen 12.1, 12.2, 12.3, 12.4, 12.5, 12.6, 12.7, 12.8 werden dann jeweils für die Probenmessung an dem unbehandelten und dem behandelten Messkörper zu den Gesamtspektren zusammengesetzt 140. Aus dem Gesamtspektrum der Messungen an dem unbehandelten Messkörper 3 und dem Gesamtspektrum der Messungen an dem mit einem Strahlenschutzmittel behandelten Messkörper 3 wird dann der Schutzfaktor des verwendeten Strahlungsschutzmittels ermittelt 150.

Nachfolgend werden Alternativen zur Durchführung der Referenzmessung 110, der Testmessung 120 und der Probenmessung 130 dargestellt.

Nach der Durchführung einer Referenzmessung 110 und einer Testmessung 120 wird ein Messspektrum mit der einen Strahlquelle 12.1 von eines nicht mit Sonnenschutz behandelten Messkörpers 3 aufgenommen 131. Das Messspektrum werden anschließend durch ein entsprechendes Softwareprogramm auf der Steuerungseinrichtung 2 mathematisch gefiltert 132, bevorzugt durch Überlagerung mit einer Trapezfunktion und/oder einer geeigneten anderen Filterfunktion. Das gefilterte Messspektrum ist derart gefiltert, dass es außerhalb des festgelegten spektralen Detektionsfensters (s. Verfahrensschritt 120) eine maximale gefilterte Intensität von 15% der maximalen ursprünglichen Intensität des Signals innerhalb des festgelegten spektralen Detektionsfensters aufweist. In alternativen Ausführungsbeispielen liegt die maximale gefilterte Intensität bei 10% oder 5 % der maximalen ursprünglichen Intensität des Signals innerhalb des festgelegten spektralen Detektionsfensters.

Zusätzlich kann nach Aufnahme 131 und Filterung 132 eines Messspektrums eines mit Sonnenschutz unbehandelten Messkörpers 3 einer Strahlquelle 12.1 ein Messspektrum derselben Strahlquelle 12.1 eines mit Sonnenschutz behandelten Messkörpers 3 aufgenommen werden. Aufgrund dieser Verfahrensschritte 131, 132 wird bei der eigentlichen Bestimmung des Sonnenschutzfaktors sowohl der Eigenschutz der Haut des Probanden als auch der Einfluss eines aufgetragenen Sonnenschutzes berücksichtigt. Dadurch kann insbesondere die Belichtungszeit während der Bestimmung des Sonnenschutzfaktors 150 reduziert werden, indem bei hohem Sonnenschutzfaktor eine höhere Intensität des auf die Probe 3 eingestrahlten Spektrums gewählt werden kann als bei niedrigem Sonnenschutzfaktor, ohne die Haut des Probanden zu schädigen. Außerdem ist durch die gezielte Ansteuerung einer oder mehrerer Strahlquellen 12.1, 12.2, 12.3, 12.4, 12.5, 12.6, 12.7, 12.8 die Untersuchung von wellenlängenabhängigen Effekten, z.B. Erkennung von Fluoreszenz, möglich.

Im nächsten Verfahrensschritt 133 erfolgt eine Abfrage, ob jede der in der Strahlquelleneinrichtung 12 verbauten Strahlquellen 12.1, 12.2, 12.3, 12.4, 12.5, 12.6, 12.7, 12.8 die Probenmessung 130 (Aufnahme des Messspektrums und Filterung) durchlaufen hat. Ist dies nicht der Fall, wird die Probenmessung 130 für eine weitere Strahlquelle 12.1, 12.2, 12.3, 12.4, 12.5, 12.6, 12.7, 12.8 wiederholt, bis jede Strahlquelle 12.1, 12.2, 12.3, 12.4, 12.5, 12.6, 12.7, 12.8 das Verfahren 130 durchlaufen hat. Hiernach erfolgt eine Abfrage 133, ob die jeweilige Aufnahme des Messspektrums 131 und die Filterung für alle vorgesehenen Lichtquellen 123 und eine Abfrage 134 ob die Aufnahme eines Messspektrums 131 und die Filterung des Messspektrums 132 sowohl für einen unbehandelten als auch einen behandelten Messkörper 3 erfolgt ist.

Im nächsten Verfahrensschritt 140 wird aus den einzelnen Emissionsspektren der einzelnen Strahlquellen 12.1, 12.2, 12.3, 12.4, 12.5, 12.6, 12.7, 12.8 ein Gesamtspektrum zusammengesetzt, das im nächsten Verfahrensschritt 150 auf die Probe 3 eingestrahlt und ein Sonnenschutzfaktor bestimmt wird.

Dazu wird üblicherweise eine Stelle an der Innenseite des Unterarms oder des Rückens eines Probanden 3 beaufschlagt. Dieser Messort 3 wird mit dem Messsystem 1 vermessen, indem Licht an einer definierten Fläche von etwa 500 µm Durchmesser - erzeugt beispielsweise durch Aufsetzen einer Lichtleitfaser 4.1 (Beleuchtungsfaser) mit einem Kerndurchmesser von 500 µm - eingestrahlt wird. Kleinere Kerndurchmesser mit beispielsweise 200 µm, 100 µm oder 50 µm sind ebenfalls möglich. Die Einstrahlung erfolgt mit einer Intensität, die keine akute Schädigung in der Haut verursacht, was unterhalb der einfachen MED, bzw. unterhalb der MZB-Werte, bzw. deutlich unterhalb den durch Sonneneinstrahlung verursachten Werten liegt. 1 MED entspricht der geringsten Bestrahlungsdosis, die bei der Ablesung nach 24 Stunden ein scharf begrenztes Erythem (Rötung) der Haut verursacht hat.

Dieses Licht durchläuft die Haut des Probanden und tritt in einem Abstand aus einer Detektionsfläche aus, die wiederum aus einer aufgesetzten Lichtleitfaser besteht.

Zur Erhöhung der Empfindlichkeit bzw. weiteren Senkung der Beleuchtungsintensität können mehrere Detektionsfasern 4.1, 4.2 in gleichem oder zumindest ähnlichem Abstand von dem Rand der Beleuchtungsfaser in einem optischen Messkopf, der in direktem Kontakt mit dem Messort 3 steht, angeordnet werden und zusammen auf eine Detektionsvorrichtung geführt und damit die Intensität gemessen werden. Abhängig von der Höhe der Intensität und dem gewählten Detektor 13 wird das durch die Strahlung erzeugte Signal um einen definierten Faktor verstärkt, der für die folgende Messung der schwächeren Intensität ebenfalls ein über dem Rauschen liegendes Signal liefert. Die Detektion erfolgt wellenlängenaufgelöst. Die Auflösung kann beispielsweise 1 nm betragen und ist abhängig von der Definition des Lichtschutzfaktors zu wählen.

Außerdem ist eine Wiederholung der Einzelmessungen während eines Messzyklus denkbar, wobei die Einzelmessungen gemittelt oder akkumuliert werden. Zudem können mehrere Messungen periodisch, beispielsweise alle 5 Sekunden, durchgeführt und analysiert werden, bis die Abweichung der aufeinanderfolgenden Werte unterhalb der einfachen Standardabweichung liegt, also stabile Werte zeigt. Eine solche Auswertung erfolgt in der Steuerungseinrichtung 2, die bei Erreichen der stabilen Werte die Messung beendet und dies einem Anwender signalisiert.

In Abwandlung eines der vorstehenden Ausführungsbeispiele ist zudem eine weitere Messung an einem anderen Messort 3 nach gleicher Art durchzuführen. Damit kann der Lichtschutzfaktor des gleichen Strahlungsschutzmittels mit gleichartigem Auftrag an verschiedenen Messorten 3 verglichen werden.

In Fig. 8 ist das Verfahren zur Aufnahme von Spektren 200 dargestellt. Hierzu wird zunächst eine Strahlquelle 12.1, 12.2, 12.3, 12.4, 12.5, 12.6, 12.7, 12.8 oder eine Strahlquellengruppe 12 aktiviert 210. Dann erfolgt die eigentliche Aufnahme des Spektrums 220. Im Anschluss daran wird die Strahlquelle 12.1, 12.2, 12.3, 12.4, 12.5, 12.6, 12.7, 12.8 oder einer Strahlquellengruppe 12 wieder deaktiviert 230. Zum Abschluss erfolgt eine Abfrage, ob die Spektren bereits für alle Strahlungsquellen 12.1, 12.2, 12.3, 12.4, 12.5, 12.6, 12.7, 12.8 aufgenommen wurden. Ist dies der Fall, so ist die Aufnahme der Spektren für diesen Zyklus abgeschlossen. Ist dies nicht der Fall, so wird die Schleife solange durchlaufen bis die Aufnahme eines Spektrums für alle Strahlungsquellen 12.1, 12.2, 12.3, 12.4, 12.5, 12.6, 12.7, 12.8 erfolgt ist.

### BEZUGSZEICHENLISTE

- 1: Messsystem
- 2: Steuerungseinrichtung
- 3: Probe/Messkörper
- 4.1, 4.2: Lichtleiter/Faserbündel
- 11: Strahlquellensteuerung
- 12: Strahlquelleneinrichtung
- 12.1, 12.2, 12.3, 12.4, 12.5, 12.6, 12.7, 12.8: Strahlquelle/LED
- 13: Detektor/Spektrometer
- 14: Linsensystem
- 14.1, 14.2, 14.3, 14.4, 14.5, 14.9: Linse
- 15: Linse
- 21: Verbindung Strahlquellensteuerung - Strahlquelleneinrichtung
- 22: Verbindung Detektor/Spektrometer - Steuereinrichtung
- 23: Verbindung Strahlquellensteuerung - Steuereinrichtung
- 24: Verbindung Strahlquellensteuerung - Detektor/Spektrometer
- 100: Verfahren zur Bestimmung von Sonnenschutzfaktoren
- 110: Referenzmessung
- 111: Aufnahme eines Referenzspektrums
- 112: Bestimmung der Zentralwellenlänge und der Filterwellenlängen
- 113: Bestimmung des mathematischen Filters
- 114: Abfrage, ob Referenzmessung für alle Strahlungsquellen erfolgt ist
- 120: Testmessung
- 121: Aufnahme eines Testspektrums
- 122: Bestimmung der Belichtungszeit und/oder der Lichtleistung
- 123: Abfrage, ob Testmessung für alle Strahlungsquellen erfolgt ist
- 124: Abfrage ob Testmessung an behandeltem und unbehandeltem Messkörper erfolgt ist
- 130: Probenmessung
- 131: Aufnahme eines Messspektrums
- 132: Mathematische Filterung des Messspektrums
- 133: Abfrage, ob Testmessung für alle Strahlungsquellen erfolgt ist
- 134: Abfrage ob Testmessung an behandeltem und unbehandeltem Messkörper erfolgt ist
- 140: Zusammensetzen eines Gesamtspektrums aus den Teilspektren der Strahlquellen
- 150: Berechnung des Sonnenschutzfaktors
- 200: Aufnahme eines Spektrums
- 210: Aktivieren einer Strahlquelle oder einer Strahlquellengruppe
- 220: Erfassen eines Spektrums
- 230: Deaktivieren einer Strahlquelle oder einer Strahlquellengruppe
- 240: Abfrage, ob eine Aufnahme eines Spektrums für jede Strahlungsquelle erfolgt ist

## Patentansprüche

1. Messsystem (1) zur Bestimmung von Sonnenschutzfaktoren von Sonnenschutzmitteln, das folgende Komponenten aufweist
- eine Strahlquelleneinrichtung (12)
wobei die Strahlquelleneinrichtung (12) zwei oder mehr separate Strahlquellen (12.1, 12.2, 12.3, 12.4, 12.5, 12.6, 12.7, 12.8) aufweist,
- ein Spektrometer (13)
- eine Steuerungseinrichtung (2)
wobei
die Wellenlängenspektren des von mindestens zwei der separaten Strahlquellen (12.1, 12.2, 12.3, 12.4, 12.5, 12.6, 12.7, 12.8) abgestrahlten Strahls verschieden sind und
die einzelnen Strahlquellen (12.1, 12.2, 12.3, 12.4, 12.5, 12.6, 12.7, 12.8) der Strahlquelleneinrichtung (12) separat ansteuerbar, puls- und regelbar sind,
wobei die Strahlquellen dynamisch ansteuerbar sind,
wobei Parameter der Strahlquellen in Abhängigkeit von Messdaten dynamisch während einer Messsequenz anpassbar sind.

2. Messsystem (1) zur Bestimmung von Sonnenschutzfaktoren von Sonnenschutzmitteln nach Anspruch 1
**dadurch gekennzeichnet, dass**
das Spektrometer (13) durch die eine Steuerungseinrichtung (2) steuerbar ist und/oder die vom Spektrometer (13) gemessenen Signale durch die eine Steuerungseinrichtung (2) verarbeitbar sind.

3. Messsystem (1) zur Bestimmung von Sonnenschutzfaktoren von Sonnenschutzmitteln nach einem oder mehreren der vorhergehenden Ansprüche
**dadurch gekennzeichnet, dass**
das Messsystem (1) eine Strahlquellensteuerung (11) aufweist, die geeignet ist zur Ansteuerung der Strahlquelleneinrichtung (12),
wobei die Strahlquellensteuerung (11) geeignet und dafür vorgesehen ist, die einzelnen Strahlquellen (12.1, 12.2, 12.3, 12.4, 12.5, 12.6, 12.7, 12.8) der Strahlquelleneinrichtung (12) separat zu steuern.

4. Messsystem (1) zur Bestimmung von Sonnenschutzfaktoren von Sonnenschutzmitteln nach Anspruch 3
**dadurch gekennzeichnet, dass**
mittels der Strahlquellensteuerung (11) die Wellenlänge, die Belichtungszeit und/oder die Intensität der von den einzelnen Strahlquellen (12.1, 12.2, 12.3, 12.4, 12.5, 12.6, 12.7, 12.8) ausgesandten Strahlung separat steuerbar ist.

5. Messsystem (1) zur Bestimmung von Sonnenschutzfaktoren von Sonnenschutzmitteln nach Anspruch 3 oder 4
**dadurch gekennzeichnet, dass**
die Strahlquellensteuerung (11) separat von der Steuerungseinrichtung (2) angeordnet ist.

6. Messsystem (1) zur Bestimmung von Sonnenschutzfaktoren von Sonnenschutzmitteln nach einem oder mehreren der Ansprüche 3 bis 5
**dadurch gekennzeichnet, dass**
die Strahlquellensteuerung (11) durch die Steuerungseinrichtung (2) steuerbar ist.

7. Messsystem (1) zur Bestimmung von Sonnenschutzfaktoren von Sonnenschutzmitteln nach einem oder mehreren der Ansprüche 3 bis 6
**dadurch gekennzeichnet, dass**
die Strahlquellensteuerung (11) mit dem Spektrometer (13) verbunden ist und geeignet und dafür vorgesehen ist, Signale des Spektrometers (13) zu empfangen und/oder Steuerbefehle an das Spektrometer (13) zu senden.

8. Verfahren (100) zur Bestimmung von Sonnenschutzfaktoren von Sonnenschutzmitteln mit Hilfe einer spektroskopischen Messung mit den Verfahrensschritten:
- Ansteuern einer ersten Strahlquelle (12.1) einer mehrere Strahlquellen (12.1, 12.2, 12.3, 12.4, 12.5, 12.6, 12.7, 12.8) aufweisenden Strahlquelleneinrichtung (12),
- Ansteuern einer zweiten Strahlquelle (12.2) einer mehrere Strahlquellen (12.1, 12.2, 12.3, 12.4, 12.5, 12.6, 12.7, 12.8) aufweisenden Strahlquelleneinrichtung (12),
- Bestrahlen eines Messkörpers (3) mit Strahlung eines Gesamtspektrums aus der ersten und der zweiten Strahlquelle (12.1, 12.2),
wobei
der Ansteuerungsbefehl der ersten Strahlquelle (12.1) verschieden ist von dem Ansteuerungsbefehl der zweiten Strahlquelle (12.2) und
die einzelnen Strahlquellen (12.1, 12.2, 12.3, 12.4, 12.5, 12.6, 12.7, 12.8) der Strahlquelleneinrichtung (12) separat ansteuerbar, puls- und regelbar sind,
wobei die Strahlquellen dynamisch ansteuerbar sind
wobei Parameter der Strahlquellen in Abhängigkeit von Messdaten dynamisch während einer Messsequenz anpassbar sind.

9. Verfahren (100) zur Bestimmung von Sonnenschutzfaktoren von Sonnenschutzmitteln mit Hilfe einer spektroskopischen Messung nach Anspruch 8
**dadurch gekennzeichnet, dass**
das Verfahren (100) das Aufnehmen (110) eines Referenzspektrums einer Strahlquelle (12.1, 12.2, 12.3, 12.4, 12.5, 12.6, 12.7, 12.8) umfasst,
wobei aus dem Referenzspektrum der einen Strahlquelle (12.1) eine Zentralwellenlänge der einen Strahlquelle (12.1) ein spektrales Detektionsfenster für die eine Strahlquelle (12.1) bestimmt (120) wird.

10. Verfahren (100) zur Bestimmung von Sonnenschutzfaktoren von Sonnenschutzmitteln mit Hilfe einer spektroskopischen Messung nach Anspruch 8 oder 9
**dadurch gekennzeichnet, dass**
das Verfahren (100) das Aufnehmen (130) eines ersten Testspektrums einer Strahlquelle (12.1, 12.2, 12.3, 12.4, 12.5, 12.6, 12.7, 12.8) umfasst,
wobei das erste Testspektrum von der Bestrahlung eines unbehandelten Messkörpers (3) aufgenommen (130) wird.

11. Verfahren (100) zur Bestimmung von Sonnenschutzfaktoren von Sonnenschutzmitteln mit Hilfe einer spektroskopischen Messung nach einem oder mehreren der Ansprüche 8 bis 10
**dadurch gekennzeichnet, dass**
das Verfahren (100) das Aufnehmen eines zweiten Testspektrums einer Strahlquelle (12.1, 12.2, 12.3, 12.4, 12.5, 12.6, 12.7, 12.8) umfasst,
wobei das zweite Testspektrum von der Bestrahlung eines mit einem Sonnenschutzmittel behandelten Messkörpers (3) aufgenommen wird.

12. Verfahren (100) zur Bestimmung von Sonnenschutzfaktoren von Sonnenschutzmitteln mit Hilfe einer spektroskopischen Messung nach einem oder mehreren der Ansprüche 8 bis 11
**dadurch gekennzeichnet, dass**
das Verfahren (100) das mathematische Filtern (140) des mit einer Strahlquelle gemessenen Remissionsspektrums (12.1, 12.2, 12.3, 12.4, 12.5, 12.6, 12.7, 12.8) umfasst,
wobei das mathematische Filtern (140) des Spektrums einer Strahlquelle (12.1, 12.2, 12.3, 12.4, 12.5, 12.6, 12.7, 12.8) derart erfolgt, dass das Signal eines gefilterten Spektrums außerhalb des festgelegten spektralen Fensters der einen Strahlquelle (12.1, 12.2, 12.3, 12.4, 12.5, 12.6, 12.7, 12.8) eine gefilterte maximale Intensität von 15% der maximalen ursprünglichen Intensität des Signals innerhalb des festgelegten spektralen Fensters aufweist.

13. Verfahren (100) zur Bestimmung von Sonnenschutzfaktoren von Sonnenschutzmitteln mit Hilfe einer spektroskopischen Messung nach einem oder mehreren der Ansprüche 8 bis 12
**dadurch gekennzeichnet, dass**
einer oder mehrere der Verfahrensschritte (110, 120, 130, 140) gemäß Anspruch 9 bis 13 für jede Strahlquelle (12.1, 12.2, 12.3, 12.4, 12.5, 12.6, 12.7, 12.8) separat ausgeführt wird.

14. Verfahren (100) zur Bestimmung von Sonnenschutzfaktoren von Sonnenschutzmitteln mit Hilfe einer spektroskopischen Messung nach einem oder mehreren der Ansprüche 8 bis 13
**dadurch gekennzeichnet, dass**
das Verfahren (100) das Zusammensetzen (200) eines Gesamtspektrums aus den gefilterten Spektren von zwei oder mehr Strahlquellen (12.1, 12.2, 12.3, 12.4, 12.5, 12.6, 12.7, 12.8) umfasst,
wobei das Verfahren (100) das Zusammensetzen (200) eines ersten Gesamtspektrums aus gefilterten Spektren aus den ersten Messspektren von zwei oder mehr Strahlquellen (12.1, 12.2, 12.3, 12.4, 12.5, 12.6, 12.7, 12.8) und/oder das Zusammensetzen (200) eines zweiten Gesamtspektrums aus gefilterten Spektren aus den zweiten Messspektren von zwei oder mehr Strahlquellen (12.1, 12.2, 12.3, 12.4, 12.5, 12.6, 12.7, 12.8) umfasst.

## Claims

1. A measurement system (1) for determining the sun protection factors of sunscreens, comprising the following components
- a radiation source device (12)
wherein the radiation source device (12) comprises two or more separate radiation sources (12.1, 12.2, 12.3, 12.4, 12.5, 12.6, 12.7, 12.8),
- a spectrometer (13)
- a control device (2)
wherein the wavelength spectra of the beams emitted by at least two of the separate beam sources (12.1, 12.2, 12.3, 12.4, 12.5, 12.6, 12.7, 12.8) are different, and
the individual beam sources (12.1, 12.2, 12.3, 12.4, 12.5, 12.6, 12.7, 12.8) of the beam source device (12) can be controlled separately, pulsed, and regulated,
wherein the beam sources can be dynamically controlled,
wherein parameters of the radiation sources can be dynamically adjusted during a measurement sequence based on measurement data.

2. Measurement system (1) for determining sun protection factors of sunscreens according to claim 1
**characterized in that**
the spectrometer (13) is controllable by the control device (2) and/or the signals measured by the spectrometer (13) are processable by the control device (2).

3. Measuring system (1) for determining sun protection factors of sunscreens according to one or more of the preceding claims
**characterized in that**
the measuring system (1) comprises a radiation source controller (11) that is suitable for controlling the radiation source device (12),
wherein the radiation source controller (11) is suitable and designed to control the individual radiation sources (12.1, 12.2, 12.3, 12.4, 12.5, 12.6, 12.7, 12.8) of the radiation source device (12) separately.

4. Measuring system (1) for determining sun protection factors of sunscreens according to claim 3
**characterized in that**
the wavelength, the exposure time, and/or the intensity of the radiation emitted by the individual radiation sources (12.1, 12.2, 12.3, 12.4, 12.5, 12.6, 12.7, 12.8) can be controlled separately by means of the radiation source controller (11).

5. A measurement system (1) for determining sun protection factors of sunscreens according to claim 3 or 4
**characterized in that**
the radiation source control unit (11) is arranged separately from the control device (2).

6. Measuring system (1) for determining sun protection factors of sunscreens according to one or more of claims 3 through 5
**characterized in that**
the radiation source controller (11) is controllable by the control device (2).

7. A measurement system (1) for determining sun protection factors of sunscreens according to one or more of claims 3 through 6
**characterized in that**
the radiation source controller (11) is connected to the spectrometer (13) and is suitable and designed to receive signals from the spectrometer (13) and/or to send control commands to the spectrometer (13).

8. A method (100) for determining sun protection factors of sunscreens using a spectroscopic measurement, comprising the following method steps:
- controlling a first radiation source (12.1) of a radiation source device (12) comprising a plurality of radiation sources (12.1, 12.2, 12.3, 12.4, 12.5, 12.6, 12.7, 12.8),
- controlling a second radiation source (12.2) of a radiation source device (12) comprising a plurality of radiation sources (12.1, 12.2, 12.3, 12.4, 12.5, 12.6, 12.7, 12.8),
- irradiating a test specimen (3) with radiation of a combined spectrum from the first and second radiation sources (12.1, 12.2),
wherein the control command for the first radiation source (12.1) differs from the control command for the second radiation source (12.2), and
the individual radiation sources (12.1, 12.2, 12.3, 12.4, 12.5, 12.6, 12.7, 12.8) of the radiation source assembly (12) can be controlled separately, pulsed, and regulated,
wherein the beam sources can be dynamically controlled
wherein parameters of the light sources can be dynamically adjusted during a measurement sequence based on measurement data.

9. A method (100) for determining sun protection factors of sunscreens using a spectroscopic measurement according to claim 8
**characterized in that**
the method (100) comprises recording (110) a reference spectrum of a radiation source (12.1, 12.2, 12.3, 12.4, 12.5, 12.6, 12.7, 12.8),
wherein a central wavelength of the single light source (12.1) and a spectral detection window for the single light source (12.1) are determined (120) from the reference spectrum of the single light source (12.1).

10. Method (100) for determining sun protection factors of sunscreens using a spectroscopic measurement according to claim 8 or 9
**characterized in that**
the method (100) comprises recording (130) a first test spectrum of a radiation source (12.1, 12.2, 12.3, 12.4, 12.5, 12.6, 12.7, 12.8),
wherein the first test spectrum is recorded (130) by irradiating an untreated test specimen (3).

11. A method (100) for determining sun protection factors of sunscreens using a spectroscopic measurement according to one or more of claims 8 through 10
**characterized in that**
the method (100) comprises recording a second test spectrum from a radiation source (12.1, 12.2, 12.3, 12.4, 12.5, 12.6, 12.7, 12.8),
wherein the second test spectrum is recorded from the irradiation of a test specimen (3) treated with a sunscreen product.

12. Method (100) for determining sun protection factors of sunscreens using a spectroscopic measurement according to one or more of claims 8 through 11
**characterized in that**
the method (100) comprises mathematically filtering (140) the remission spectrum (12.1, 12.2, 12.3, 12.4, 12.5, 12.6, 12.7, 12.8) measured with a radiation source,
wherein the mathematical filtering (140) of the spectrum of a radiation source (12.1, 12.2, 12.3, 12.4, 12.5, 12.6, 12.7, 12.8) is performed such that the signal of a filtered spectrum outside the specified spectral window of the beam source (12.1, 12.2, 12.3, 12.4, 12.5, 12.6, 12.7, 12.8) has a filtered maximum intensity of 15% of the maximum original intensity of the signal within the specified spectral window.

13. A method (100) for determining sun protection factors of sunscreens using a spectroscopic measurement according to one or more of claims 8 through 12
**characterized in that**
one or more of the method steps (110, 120, 130, 140) according to claims 9 through 13 are performed separately for each radiation source (12.1, 12.2, 12.3, 12.4, 12.5, 12.6, 12.7, 12.8).

14. A method (100) for determining sun protection factors of sunscreens using a spectroscopic measurement according to one or more of claims 8 through 13
**characterized in that**
the method (100) comprises combining (200) a composite spectrum from the filtered spectra of two or more radiation sources (12.1, 12.2, 12.3, 12.4, 12.5, 12.6, 12.7, 12.8),
wherein the method (100) comprises combining (200) a first composite spectrum from filtered spectra derived from the first measurement spectra of two or more radiation sources (12.1, 12.2, 12.3, 12.4, 12.5, 12.6, 12.7, 12.8) and/or combining (200) a second composite spectrum from filtered spectra derived from the second measurement spectra of two or more radiation sources (12.1, 12.2, 12.3, 12.4, 12.5, 12.6, 12.7, 12.8).

## Revendications

1. Système de mesure (1) pour déterminer les facteurs de protection de crèmes solaires, qui présente les composants suivants :
- un dispositif de source de rayonnement (12)
dans lequel le dispositif de source de rayonnement (12) présente deux sources de rayonnement distinctes (12.1, 12.2, 12.3, 12.4, 12.5, 12.6, 12.7, 12.8) ou plus,
- un spectromètre (13)
- un dispositif de commande (2)
dans lequel les spectres de longueur d'onde du faisceau émis par au moins deux des sources de faisceau distinctes (12.1, 12.2, 12.3, 12.4, 12.5, 12.6, 12.7, 12.8) sont différents et
les différentes sources de rayonnement (12.1, 12.2, 12.3, 12.4, 12.5, 12.6, 12.7, 12.8) du dispositif de source de rayonnement (12) peuvent être contrôlées séparément, pulsées et régulées,
dans lequel les sources de rayonnement peuvent être contrôlées de manière dynamique,
dans lequel des paramètres des sources de rayonnement peuvent être adaptés dynamiquement au cours d'une séquence de mesure en fonction de données de mesure.

2. Système de mesure (1) pour déterminer les facteurs de protection de crèmes solaires selon la revendication 1
**caractérisé en ce que**
le spectromètre (13) peut être commandé par l'un dispositif de commande (2) et/ou les signaux mesurés par le spectromètre (13) peuvent être traités par l'un dispositif de commande (2).

3. Système de mesure (1) pour déterminer les facteurs de protection de crèmes solaires selon l'une ou plusieurs des revendications précédentes
**caractérisé en ce que**
le système de mesure (1) présente une unité de commande des sources de rayonnement (11), apte à contrôler le dispositif de source de rayonnement (12),
dans lequel l'unité de commande des sources de rayonnement (11) est apte et prévue pour commander séparément les différentes sources de rayonnement (12.1, 12.2, 12.3, 12.4, 12.5, 12.6, 12.7, 12.8) du dispositif de source de rayonnement (12).

4. Système de mesure (1) pour déterminer les facteurs de protection de crèmes solaires selon la revendication 3
**caractérisé en ce que**
au moyen de l'unité de commande des sources de rayonnement (11), la longueur d'onde, la durée d'exposition et/ou l'intensité du rayonnement émis par chacune des sources de rayonnement (12.1, 12.2, 12.3, 12.4, 12.5, 12.6, 12.7, 12.8) peuvent être commandées séparément.

5. Système de mesure (1) pour déterminer les facteurs de protection de crèmes solaires selon la revendication 3 ou 4
**caractérisé en ce que**
l'unité de commande des sources de rayonnement (11) est disposée séparément du dispositif de commande (2).

6. Système de mesure (1) pour déterminer les facteurs de protection de crèmes solaires selon l'une ou plusieurs des revendications 3 à 5
**caractérisé en ce que**
l'unité de commande des sources de rayonnement (11) peut être commandée par le dispositif de commande (2).

7. Système de mesure (1) pour déterminer les facteurs de protection de crèmes solaires selon l'une ou plusieurs des revendications 3 à 6
**caractérisé en ce que**
l'unité de commande des sources de rayonnement (11) est reliée au spectromètre (13) et est apte et prévue pour recevoir des signaux du spectromètre (13) et/ou envoyer des instructions de commande au spectromètre (13).

8. Procédé (100) pour déterminer les facteurs de protection de crèmes solaires à l'aide d'une mesure spectroscopique, comprenant les étapes de procédé :
- de contrôle d'une première source de rayonnement (12.1) d'un dispositif de source de rayonnement (12) présentant plusieurs sources de rayonnement (12.1, 12.2, 12.3, 12.4, 12.5, 12.6, 12.7, 12.8),
- de contrôle d'une seconde source de rayonnement (12.2) d'un dispositif de source de rayonnement (12) présentant plusieurs sources de rayonnement (12.1, 12.2, 12.3, 12.4, 12.5, 12.6, 12.7, 12.8),
- d'irradiation d'un corps de mesure (3) au moyen d'un rayonnement d'un spectre global provenant de la première et de la seconde source de rayonnement (12.1, 12.2),
dans lequel l'instruction de contrôle de la première source de rayonnement (12.1) est différente de l'instruction de contrôle de la seconde source de rayonnement (12.2) et
les différentes sources de rayonnement (12.1, 12.2, 12.3, 12.4, 12.5, 12.6, 12.7, 12.8) du dispositif de source de rayonnement (12) peuvent être contrôlées séparément, pulsées et régulées,
dans lequel les sources de rayonnement peuvent être contrôlées de manière dynamique
dans lequel des paramètres des sources de rayonnement peuvent être adaptés dynamiquement au cours d'une séquence de mesure en fonction de données de mesure.

9. Procédé (100) pour déterminer les facteurs de protection de crèmes solaires à l'aide d'une mesure spectroscopique selon la revendication 8
**caractérisé en ce que**
le procédé (100) comprend l'acquisition (110) d'un spectre de référence d'une source de rayonnement (12.1, 12.2, 12.3, 12.4, 12.5, 12.6, 12.7, 12.8),
dans lequel une longueur d'onde centrale de l'une source de rayonnement (12.1) et une fenêtre de détection spectrale de l'une source de rayonnement (12.1) sont déterminées (120) à partir du spectre de référence pour l'une source de rayonnement (12.1).

10. Procédé (100) pour déterminer les facteurs de protection de crèmes solaires à l'aide d'une mesure spectroscopique selon la revendication 8 ou 9
**caractérisé en ce que**
le procédé (100) comprend l'acquisition (130) d'un premier spectre d'essai d'une source de rayonnement (12.1, 12.2, 12.3, 12.4, 12.5, 12.6, 12.7, 12.8),
dans lequel le premier spectre d'essai est acquis (130) à partir de l'irradiation d'un corps de mesure (3) non traité.

11. Procédé (100) pour déterminer les facteurs de protection de crèmes solaires à l'aide d'une mesure spectroscopique selon l'une ou plusieurs des revendications 8 à 10
**caractérisé en ce que**
le procédé (100) comprend l'acquisition d'un second spectre d'essai d'une source de rayonnement (12.1, 12.2, 12.3, 12.4, 12.5, 12.6, 12.7, 12.8),
dans lequel le second spectre d'essai est acquis à partir de l'irradiation d'un corps de mesure (3) traité avec une crème solaire.

12. Procédé (100) pour déterminer les facteurs de protection de crèmes solaires à l'aide d'une mesure spectroscopique selon l'une ou plusieurs des revendications 8 à 11
**caractérisé en ce que**
le procédé (100) comprend le filtrage mathématique (140) du spectre de rémission mesuré au moyen d'une source de rayonnement (12.1, 12.2, 12.3, 12.4, 12.5, 12.6, 12.7, 12.8),
dans lequel le filtrage mathématique (140) du spectre d'une source de rayonnement (12.1, 12.2, 12.3, 12.4, 12.5, 12.6, 12.7, 12.8) est réalisé de sorte que le signal d'un spectre filtré présente, en dehors de la fenêtre spectrale définie de l'une source de rayonnement (12.1, 12.2, 12.3, 12.4, 12.5, 12.6, 12.7, 12.8), une intensité maximale filtrée égale à 15 % de l'intensité maximale initiale du signal à l'intérieur de la fenêtre spectrale définie.

13. Procédé (100) pour déterminer les facteurs de protection de crèmes solaires à l'aide d'une mesure spectroscopique selon l'une ou plusieurs des revendications 8 à 12
**caractérisé en ce que**
l'une ou plusieurs des étapes de procédé (110, 120, 130, 140) selon les revendications 9 à 13 sont exécutées séparément pour chaque source de rayonnement (12.1, 12.2, 12.3, 12.4, 12.5, 12.6, 12.7, 12.8).

14. Procédé (100) pour déterminer les facteurs de protection de crèmes solaires à l'aide d'une mesure spectroscopique selon l'une ou plusieurs des revendications 8 à 13
**caractérisé en ce que**
le procédé (100) comprend l'assemblage (200) d'un spectre global à partir des spectres filtrés de deux sources de rayonnement (12.1, 12.2, 12.3, 12.4, 12.5, 12.6, 12.7, 12.8) ou plus,
dans lequel le procédé (100) comprend l'assemblage (200) d'un premier spectre global à partir de spectres filtrés issus des premiers spectres de mesure de deux sources de rayonnement (12.1, 12.2, 12.3, 12.4, 12.5, 12.6, 12.7, 12.8) ou plus et/ou l'assemblage (200) d'un second spectre global à partir de spectres filtrés issus des seconds spectres de mesure de deux sources de rayonnement (12.1, 12.2, 12.3, 12.4, 12.5, 12.6, 12.7, 12.8) ou plus.
